# EUROPEAN PATENT SPECIFICATION

(11) **EP 3 995 206 B1**
(45) Date of publication and mention of the grant of the patent: **30.08.2023**
(21) Application number: 19937266.5
(22) Date of filing: 05.07.2019
(51) Int. Cl.: B01J 13/14, A61L 27/12, A61L 27/54, A61K 8/24, A61K 8/72, C08J 3/24

(54) **PREPARATION METHOD OF A DRUG-CARRYING MICROSPHERE**
VERFAHREN ZUR HERSTELLUNG EINER ARZNEIMITTELTRAGENDEN MIKROKUGEL
PROCÉDÉ DE PRÉPARATION D'UNE MICROSPHÈRE TRANSPORTANT UN MÉDICAMENT

(43) Date of publication of application: 11.05.2022
(73) Proprietor: Guangzhou Zhongda Medical Instrument Co., Ltd., Guangzhou, Guangdong 510000 (CN)
(72) Inventor: YANG, Yuexiong, Guangzhou, Guangdong 510000 (CN); ZHANG, Chao, Guangzhou, Guangdong 510000 (CN); YANG, Yang, Guangzhou, Guangdong 510000 (CN); ZHANG, Yang, Guangzhou, Guangdong 510000 (CN); HUANG, Dan, Guangzhou, Guangdong 510000 (CN); LIU, Jingling, Guangzhou, Guangdong 510000 (CN); LI, Jing, Guangzhou, Guangdong 510000 (CN); WU, Jiexin, Guangzhou, Guangdong 510000 (CN)
(74) Representative: Sun, Yiming
(86) International application number: PCT/CN2019/094868
(87) International publication number: WO 2021/003601

(56) References cited:
- CN-A- 101 822 961
- CN-A- 107 376 795
- CN-A- 107 899 071
- PIETRZYNSKA MONIKA ET AL: "Poly(vinyl alcohol)/hydroxyapatite Monolithic In-Needle Extraction (MINE) device: Preparation and examination of drug affinity", EUROPEAN JOURNAL OF PHARMACEUTICAL SCIENCES, ELSEVIER AMSTERDAM, NL, vol. 105, 17 May 2017 (2017-05-17), pages 195-202, XP085069252, ISSN: 0928-0987, DOI: 10.1016/J.EJPS.2017.05.040
- CASTILLO SERGIO ANDRES PINEDA ET AL: "Novel biopolimeric system for bone tissue engineering: crosslinked and plasticized chitosan/poly vinyl alcohol/hydroxiapatite scaffolds", 2018 IX INTERNATIONAL SEMINAR OF BIOMEDICAL ENGINEERING (SIB), IEEE, 16 May 2018 (2018-05-16), pages 1-7, XP033404935, DOI: 10.1109/SIB.2018.8467728 [retrieved on 2018-09-18]
- Guo Jiahu , Li li , Zhou Yanming: "Preparation of Crosslinked Mircospheres of CPVA with Inverse Suspension-Chemical Crosslinking Method", Shanghai Chemical Industry, vol. 36, no. 3, 15 March 2011 (2011-03-15) , pages 20-23, XP055883783, ISSN: 1004-017x, DOI: 10.16759/j.cnki.issn.1004-017x.2011.03.011
- Dong Jiaqun: "Synthesis And Properties of Polyvinyl Alcohol Microspheres", China Master s Theses Full-Text Database, no. 2, 15 February 2016 (2016-02-15), XP009532999,

## Description

### FIELD OF THE INVENTION

The present invention relates to a method of preparing microspheres capable of drug-loading.

### BACKGROUND OF THE INVENTION

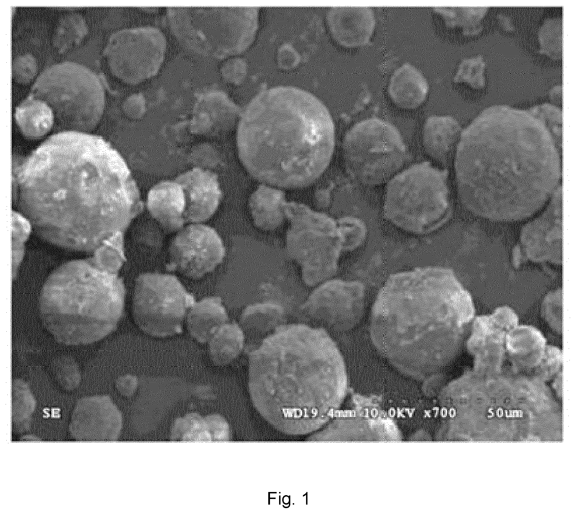

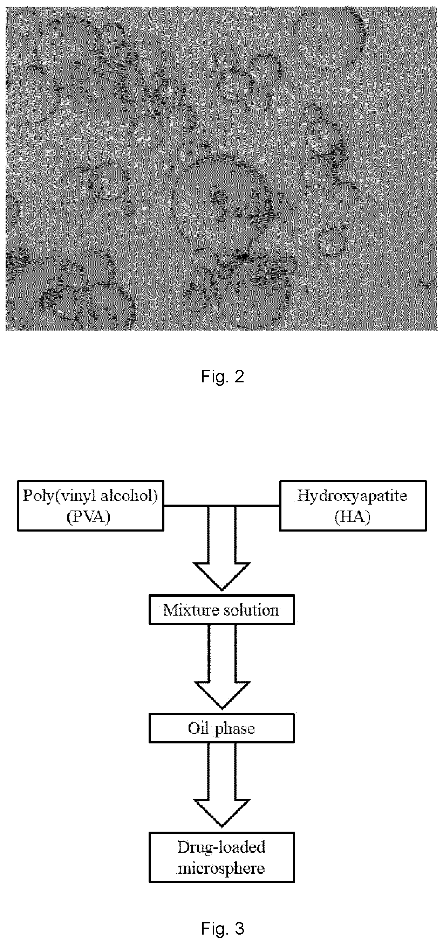

Microspheres can be used as drug carriers or as interventional embolization materials in medical treatment. Common microspheres capable of drug-loading include poly(vinyl alcohol) (PVA) microspheres, gelatin microspheres, sodium alginate microspheres, poly(lactic acid) microspheres, and albumin microspheres, etc. For example, liver cancer can be treated by interventional methods in clinical treatment, and the interventional material is embolization microspheres. Microspheres have two functions: First, the microspheres block blood vessel in the target tumor blood vessels and block the source of nutrition for tumor cells. Second, the microspheres can carry drugs, and the released drug can inhibit tumor growth. The patent application titled "A Preparation Method of Poly(vinyl alcohol)/Hydroxyapatite Composite Microspheres" (Application No. CN201710584555.9) presents a method in which the pH of the reaction mixture was adjusted using an alkaline solution to allow the in-situ deposition of hydroxyapaptite (HA) after adding glutaraldehyde as cross-linker to the PVA; the as-prepared microspheres were then centrifuged, washed, and air-dried to obtain PVA/HA composite microspheres. The scanning electron microscopic image of the microspheres is shown in Figure 1. The microspheres are inconsistent in size with rough surfaces. A 500× microscopic image of the microspheres is shown in Figure 2. The microspheres are semi-transparent and have no radiopaque effect under X-ray irradiation. Document CN 107376795 A discloses a method to produce hydroxyapatite coated with crosslinked PVA.

In the prior art, the preparation methods of microspheres generally include reverse suspension polymerization, electrostatic spraying, emulsification and cross-linking, phase separation, solvent evaporation, and single emulsion. The methods described in the prior art are introduced as follows.

In reverse suspension polymerization, the oil phase is used as the continuous phase, the aqueous phase is used as the dispersed phase, a certain amount of catalyst and water-soluble initiator are added to the aqueous phase, and a surfactant is added to the oil phase. The PVA in the aqueous phase is cross-linked by the added water-soluble initiator to form a cross-linked product.

The electrostatic spray method is: applying high voltage to the polymer solution during the electrostatic spraying process, and the polymer solution is charged under the action of the electrostatic field and forms hemispherical droplets at the tip of the spinneret. When the intensity of the electric field reaches the critical value, the solution is sprayed from the tip, and the molecular chains of the low-concentration polymer solution form microspheres under the combined action of the electric field and surface tension due to the low degree of entanglement.

The emulsification and cross-linking method employs continuous stirring and other methods to add a cross-linker to the stable emulsion. Since the reactive aldehyde group in the cross-linker can undergo condensation/addition reaction with the amino group or alcohol group of the polymer material, microspheres are made by cross-linking.

The phase separation method is adding a non-solvent substance or inorganic salt as a coagulant in the mixed solution of the drug and the polymer carrier, thereby reducing the solubility of the polymer, thus the polymer is precipitated in the mixed solution and wrapped on the surface of the drug. After curing the protective layer through a series of methods, microspheres with high encapsulation efficiency can be obtained.

The solvent evaporation method removes the volatile solvent of the dispersed phase from the emulsion droplets to prepare microspheres and can control the particle size of the microspheres in the nanometer range. The common solvent evaporation method is to prepare Oil/Water, Water/Oil, Water/Oil/Water and other single-emulsified and double-emulsified emulsion systems according to the properties of polymers and drugs. After a stable emulsion system is formed, methods such as heating and continuous stirring are used to diffuse the organic solvent into the interface between the continuous phase and the air to evaporate. After a series of operations such as filtration, washing, and drying, microspheres capable of drug-loading are obtained.

The single emulsion method utilizes the oil-in-water phase system. The polymer and the drug are dissolved or suspended in an organic solvent, and then emulsified with the immiscible aqueous phase to form an Oil/Water emulsion. When the organic solvent evaporates, the polymer-encapsulated drug solidifies to form the required microspheres capable of drug-loading. Generally, the loaded drugs are insoluble in water.

Among the above-mentioned methods in the prior art, the electrostatic spray method is not suitable for temperature-sensitive and heat-labile protein poly(peptide) microspheres, and it is also difficult to control the particle size of the microsphere particles. The emulsification and cross-linking method take a long time in the preparation process of microspheres, and are easily affected by the environmental factors and unsuitable for the large-scale preparation of microspheres, and the cross-linker has certain toxicity as well.

The phase separation method requires the use of a large amount of organic solvents in the preparation process, but these solvents are ultimately difficult to be removed from the microspheres and thus bring certain toxicity. Therefore, this method is easily affected by the addition of coagulants and solvent residues. The solvent evaporation method is affected by temperature. If the temperature is too low, it will prolong the production time of the microspheres and reduce the encapsulation efficiency; if the temperature is too high, the drug encapsulation will be unsatisfactory, and the controlled release performance of the microsphere matrix is poor, and the burst release of the microspheres is severe, and the particle size of the microspheres will become larger.

Pietrzynska et al., European Journal of Pharmaceutical Sciences 105, 2017, 195-202, discloses polymer-ceramic materials based on poly(vinyl alcohol) (PVA) and hydroxyapatite which were applied as absorption material in Monolithic In-Needle Extraction (MINE) device. The device is suitable for the examination of bisphosphonates affinity to bone and is a helpful tool in evaluation of the suitability of potential antiresorptive drugs.

Pineda et al., 2018 IX International Seminar of Biomedical Engineering, 16 May 2018, pages 1-7 discloses the preparation of porous scaffolds made of chitosan, polyvinyl alcohol and hydroxyapatite via freeze-drying method. Polymer solutions at three different volume ratios {1:1, 1:3, 3:1) and hydroxyapatite at constant mass ratio were blended. Glutaraldehyde and glycerol were added as a polymer-chain crosslinker and plasticizer, respectively. The obtained scaffolds were used to test bone proliferation and potential of bone regeneration and were characterized through FTIR, mechanical tests, cell culture and swelling tests.

There are many problems in the prior art, such as the particle size of the prepared microspheres cannot be adjusted, the production process is complex and easy to be affected by the environment, and inconvenience for mass production. There are also restrictions on the loading of temperature-sensitive drugs, the radiopaque effect is not good, the drug loading rate of the microspheres is not high, and the sustained release effect is unsatisfactory, etc. Therefore, the current microspheres mainly suffer from the problems of complicated and unstable production process and low drug loading.

### SUMMARY OF THE INVENTION

The objective of the present invention is to provide a method with high production efficiency and convenient industrial application for preparing microspheres capable of drug-loading. The microspheres capable of drug-loading prepared by the method have a good embolization efficiency, high drug loading, good drug-release performance, and good biocompatibility.

To achieve the above-mentioned objectives, the technical solution adopted by the present invention is as defined in appended claims.

In the preparation method of the microspheres capable of drug-loading of the present invention, a mixture of liquid paraffin and sorbitan fatty acid ester are used as the oil phase, and an aqueous solution of PVA and HA particles are mixed as the aqueous phase. Certain amount of concentrated hydrochloric acid is added to the aqueous phase as a catalyst, and then glutaraldehyde is added as a crosslinking agent for pre-reaction.

Here, the pre-reaction time is controlled as 3 seconds to 30 seconds. The reason is if the pre-reaction time is too long, it will affect the particle size. After pre-crosslinking, the reaction liquid is added dropwise to the oil phase, and then being stirred at a specific temperature in oil bath to continue the reaction. In the above process, cross-linking reaction occurs between PVA and glutaraldehyde. Their cross-linking is the aldol condensation reaction of hydroxyl group and aldehyde group. Under acidic conditions, one hydroxyl group reacts with one aldehyde group to form hemiacetal, which is then dehydrated and condensed with a hydroxyl group to form an ether. Since the PVA molecule contains large number of hydroxyl groups, the two are easy to form a cross-linked product with stable performance.

In the preparation method for the microspheres capable of drug-loading of the present invention, PVA is added to a certain amount of water to form an aqueous solution of PVA and mixed with HA particles, wherein the mass ratio of the PVA to water is 0.2-100-10-100. The HA particles possess the characteristics of large specific surface area, porous structure, high surface activity, etc., which can make the drug release from the internal pores tardily. When the HA particles are in solution, the hydroxyl groups on their surface appear vacant at a certain moment, and Ca²⁺ is exposed to form adsorption sites, which can be combined with the hydroxyl-, carboxyl- and other functional groups in the drug molecule, thereby effectively improving the performances of the microspheres capable of drug-loading regarding drug loading and encapsulation efficiency. Certainly, as an alternative, the hydroxyapatite particles can be nano-level hydroxyapatite or micron-level hydroxyapatite.

As a preferred embodiment of the method for preparing the microspheres capable of drug-loading of the present invention, the mass ratio of PVA to HA particles in Step (1) is 1:1~10:1. In the present invention, the mass ratio of PVA to HA particles has a direct impact on the encapsulation efficiency and sustained release effect of the microspheres. When the mass ratio of apatite particles is 1.5:1∼4:1, the microspheres capable of drug-loading finally prepared have better encapsulation efficiency and the best sustained-release effect. The particular mass ratio of PVA to HA particles are 1.5:1; or 2:1; or 2.5:1; or 3:1; or 4:1.

Further, the mass ratio of concentrated hydrochloric acid to PVA added in Step (2) is 14-100-15-100.

Further, the mass ratio of glutaraldehyde to PVA added in Step (2) is 1:50-1:20.

Further, the mass ratio of PVA to liquid paraffin in the mixture oil phase in Step (2) is 1:1-1:1.5.

Further, in Step (3), a lyophilizer or a dryer is used for fast drying, thus has a better effect on the formation of a rounded shape of the microspheres and improves the embolization effect.

The mass ratio of the above materials is obtained from thousands of experiments. Wherein, as the amount of glutaraldehyde added is larger, the coagulation speed of the PVA solution is faster, and the crosslinking speed of the reaction is faster. The mass ratio of liquid paraffin to sorbitan fatty acid ester is 50:1-100:1. Experiments are carried out in this range and it is found that when the amount of sorbitan fatty acid ester is added, the smaller the particle size of the prepared microspheres. According to the needs of preparing microspheres of different sizes, it can be controlled and adjusted.

In addition, the present invention also provides a microsphere capable of drug-loading with a high drug-loading content and a good sustained release effect manufactured according to the above method. The particle size of the microspheres capable of drug-loading is 25 µm to 800 µm.

Further, the encapsulation rate of the microspheres capable of drug-loading may reach 80%-90%, and the drug-loading content of the microspheres capable of drug-loading is 0.5%-8%.

The microspheres capable of drug-loading prepared by the method of the present invention and mainly have the following beneficial effects:
(1) High drug loading and good drug release effect
   Compared with the prior art, the preparation method of the present invention pre-mixes the PVA aqueous solution and the HA particles, and the obtained microspheres capable of drug-loading form more pores, and the surface is smooth and has a huge specific surface area, surface charge, good adhesion and transfer of bio-macromolecule drugs, high adsorption, thus significantly increase the drug loading of microspheres, and achieve the effect of long-acting drug release.
(2) A certain radiopaque effect
   The preparation method of the present invention pre-mixes HA particles and PVA in a certain ratio. The HA particles are embedded in the microsphere matrix after the polymerization reaction, thus makes them hard to escape; and since there is no alkaline solution added in Step 2, whereas there added glutaraldehyde to react for 3 seconds to 30 seconds, and then drop the pre-reaction solution into the mixture oil phase before the pre-reaction solution is condensed. Such control details allow the microspheres capable of drug-loading have a certain degree of radiopaque effect, thus improves the ability of medical diseases diagnosis.
(3) The microspheres capable of drug-loading prepared according to the present invention have good biocompatibility and reliable performance, because both HA particles and PVA have good bio-compatibility and no biological toxicity, and the microspheres capable of drug-loading have smooth surface, all the above features endow the microspheres with wide range of applications.
(4) The microspheres capable of drug-loading prepared according to the present invention have adjustable particle size, which is convenient for adjustment and control during production. The preparation process is more scientifically reasonable, there is no steps for adding alkaline solution for reaction, and the product quality and the production efficiency are all improved, thus the industrial application is convenient. The HA particles are mixed with the aqueous solution of PVA. In a certain concentration range, the more water is added, the smaller the particle size of the microspheres capable of drug-loading finally obtained. As used in medical treatment, the embolization effect is good.

### BRIEF DESCRIPTION OF THE DRAWINGS

Fig. 1 is a scanning electron micrograph of microspheres prepared in the prior art.
Fig. 2 is a photomicrograph of a microsphere prepared in the prior art.
Fig. 3 is a flow chart of the preparation method of the microspheres capable of drug-loading of the present invention.
Fig. 4 is a schematic diagram showing the comparison of the encapsulation efficiency of the PVA microspheres and the microspheres capable of drug-loading prepared by the present invention.
Fig. 5 is a schematic diagram showing the comparison of drug accumulation release curves between PVA microspheres and the microspheres capable of drug-loading prepared by the present invention.
Fig. 6 is a comparative test diagram of the radiopaque effect of various types of microspheres.
Fig. 7 is a scanning electron micrograph of the microspheres capable of drug-loading according to an embodiment of the present invention.
Fig. 8 is a photomicrograph of the microspheres capable of drug-loading according to an embodiment of the present invention.

### DETAILED DESCRIPTION OF THE INVENTION

In order to better illustrate the objectives, technical solutions and beneficial effects of the present invention, the present invention will be further described below with reference to the accompanying drawings and specific embodiments.

### Example 1

The preparation method of the microspheres capable of drug-loading of the present invention, is shown in Fig. 3, the preparation method includes the following steps:
(1) Mix the aqueous solution of PVA with HA particles evenly; wherein the mass ratio of PVA to water is 1:100;
(2) Add concentrated hydrochloric acid to the evenly mixture solution in Step (1), the mass ratio of concentrated hydrochloric acid to PVA is 14:100-15:100, and then glutaraldehyde is added to obtain a pre-reaction solution. After 3 seconds for pre-reaction, the pre-reaction solution is added dropwise to the mixture oil phase before the pre-reaction solution is coagulated, and the reaction is continued for at least 3 hours with stirring in an oil bath at 50°C-60°C; the mixture oil phase includes liquid paraffin and sorbitan fatty acid ester;
(3) Collect the reaction mixture in Step (2), and filter, wash, and freeze-dry to obtain microspheres capable of drug-loading;

In the present Example 1, the mass ratio of PVA to HA particles in Step (1) is 3:1; the mass ratio of glutaraldehyde to PVA added in Step (2) is 1:40, the mass ratio of PVA to liquid paraffin in the mixture oil phase in Step (2) is 1:1, and the mass ratio of liquid paraffin to sorbitan fatty acid ester in the mixture oil phase is 90:1.

### Example 2

The preparation method of the microspheres capable of drug-loading of the present invention includes the following steps:
(1) Mix the aqueous solution of PVA with HA particles evenly; wherein the mass ratio of PVA to water is 2:100;
(2) Add concentrated hydrochloric acid to the evenly mixture solution in Step (1), the mass ratio of concentrated hydrochloric acid to PVA is 14:100-15:100, and then glutaraldehyde is added to obtain a reaction solution. After 10 seconds for pre-reaction, the pre-reaction solution is added dropwise to the mixture oil phase before the pre-reaction solution is coagulated, and the reaction is continued for at least 4 hours with stirring in an oil bath at 50°C-60°C; the mixture oil phase includes liquid paraffin and sorbitan fatty acid ester;
(3) Collect the reaction mixture in Step (2), and filter, wash, and freeze-dry to obtain microspheres capable of drug-loading;

In the present Example 2, the mass ratio of PVA to HA particles in Step (1) is 2:1 (PVA:HA=2:1); the mass ratio of glutaraldehyde to PVA added in Step (2) is 1:50, the mass ratio of PVA to liquid paraffin in the mixture oil phase in Step (2) is 1:1.5, and the mass ratio of liquid paraffin to sorbitan fatty acid ester in the mixture oil phase is 80:1.

### Example 3

The preparation method of the microspheres capable of drug-loading of the present invention includes the following steps:
(1) Mix the aqueous solution of PVA with HA particles evenly; wherein the mass ratio of PVA to water is 3:100;
(2) Add concentrated hydrochloric acid to the evenly mixture solution in Step (1), the mass ratio of concentrated hydrochloric acid to PVA is 14:100-15:100, and then glutaraldehyde is added to obtain a reaction solution. After 30 seconds for pre-reaction, the pre-reaction solution is added dropwise to the mixture oil phase before the pre-reaction solution is coagulated, and the reaction is continued for at least 3 hours with stirring in an oil bath at 50°C-60°C; the mixture oil phase includes liquid paraffin and sorbitan fatty acid ester;
(3) Collect the reaction mixture in Step (2), and filter, wash, and freeze-dry to obtain microspheres capable of drug-loading;

In the present Example 3, the mass ratio of PVA to HA particles in Step (1) is 4:1 (PVA:HA=4:1); the mass ratio of glutaraldehyde to PVA added in Step (2) is 1:45, the mass ratio of PVA to liquid paraffin in the mixture oil phase in Step (2) is 1:1.2, and the mass ratio of liquid paraffin to sorbitan fatty acid ester in the mixture oil phase is 85:1.

### Example 4

The preparation method of the microspheres capable of drug-loading of the present invention includes the following steps:
(1) Mix the aqueous solution of PVA with HA particles evenly; wherein the mass ratio of PVA to water is 4:100;
(2) Add concentrated hydrochloric acid to the evenly mixture solution in Step (1), the mass ratio of concentrated hydrochloric acid to PVA is 14:100-15:100, and then glutaraldehyde is added to obtain a reaction solution. After 20 seconds for pre-reaction, the pre-reaction solution is added dropwise to the mixture oil phase before the pre-reaction solution is coagulated, and the reaction is continued for at least 3 hours with stirring in an oil bath at 50°C-60°C; the mixture oil phase includes liquid paraffin and sorbitan fatty acid ester;
(3) Collect the reaction mixture in Step (2), and filter, wash, and dry with a drier to obtain microspheres capable of drug-loading;

In the present Example 4, the mass ratio of PVA to HA particles in Step (1) is 1.5:1 (PVA:HA=1.5:1); the mass ratio of glutaraldehyde to PVA added in Step (2) is 1:20, the mass ratio of PVA to liquid paraffin in the mixture oil phase in Step (2) is 1:1, and the mass ratio of liquid paraffin to sorbitan fatty acid ester in the mixture oil phase is 50:1.

### Example 5

The preparation method of the microspheres capable of drug-loading of the present invention includes the following steps:
(1) Mix the aqueous solution of PVA with HA particles evenly; wherein the mass ratio of PVA to water is 6:100;
(2) Add concentrated hydrochloric acid to the evenly mixture solution in Step (1), the mass ratio of concentrated hydrochloric acid to PVA is 14:100-15:100, and then glutaraldehyde is added to obtain a reaction solution. After 30 seconds for pre-reaction, the pre-reaction solution is added dropwise to the mixture oil phase before the pre-reaction solution is coagulated, and the reaction is continued for at least 3 hours with stirring in an oil bath at 50°C-60°C; the mixture oil phase includes liquid paraffin and sorbitan fatty acid ester;
(3) Collect the reaction mixture in Step (2), and filter, wash, and dry with a drier to obtain microspheres capable of drug-loading;

In the present Example 4, the mass ratio of PVA to HA particles in Step (1) is 1:1 (PVA:HA=1:1); the mass ratio of glutaraldehyde to PVA added in Step (2) is 1:30, the mass ratio of PVA to liquid paraffin in the mixture oil phase in Step (2) is 1:1.3, and the mass ratio of liquid paraffin to sorbitan fatty acid ester in the mixture oil phase is 60:1.

### Example 6

The preparation method of the microspheres capable of drug-loading of the present invention includes the following steps:
(1) Mix the aqueous solution of PVA with HA particles evenly; wherein the mass ratio of PVA to water is 10:100;
(2) Add concentrated hydrochloric acid to the evenly mixture solution in Step (1), the mass ratio of concentrated hydrochloric acid to PVA is 14:100-15:100, and then glutaraldehyde is added to obtain a reaction solution. After 30 seconds for pre-reaction, the pre-reaction solution is added dropwise to the mixture oil phase before the pre-reaction solution is coagulated, and the reaction is continued for at least 3 hours with stirring in an oil bath at 50°C-60°C; the mixture oil phase includes liquid paraffin and sorbitan fatty acid ester;
(3) Collect the reaction mixture in Step (2), and filter, wash, and dry with a drier to obtain microspheres capable of drug-loading;

In the present Example 6, the mass ratio of PVA to HA particles in Step (1) is 10:1 (PVA:HA=10:1); the mass ratio of glutaraldehyde to PVA added in Step (2) is 1:25, the mass ratio of PVA to liquid paraffin in the mixture oil phase in Step (2) is 1:1.4, and the mass ratio of liquid paraffin to sorbitan fatty acid ester in the mixture oil phase is 100:1. The comparison test of the encapsulation efficiency and sustained release effect of the microspheres capable of drug-loading of the present invention is as follows

In this embodiment, a control group and a test group are set. The control group uses surface-functionalized PVA microspheres that are common in the prior art. The test group 1 uses the microspheres capable of drug-loading described in Example 2 of the present invention, and the test group 2 uses the microspheres capable of drug-loading described in Example 3 of the present invention.

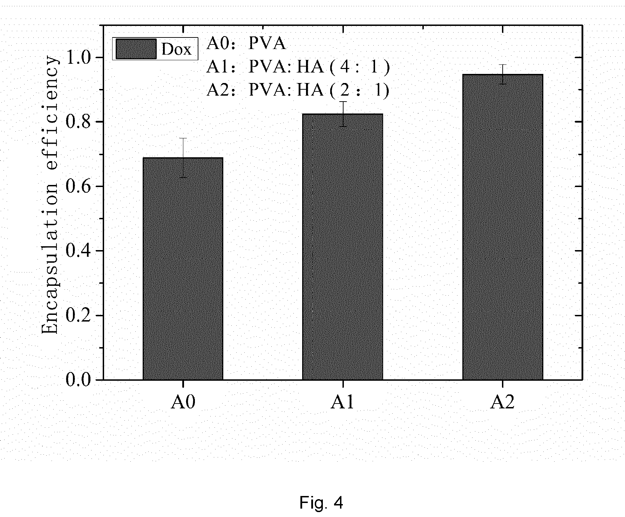

The microspheres of the control group and the test group were used to evaluate the encapsulation efficiency of the microspheres respectively. The encapsulation ratio refers to the percentage of the amount of drug packaged into the microspheres to the total amount of drug input. Encapsulation rate = (W₁/W₂)×100%, where W₁ refers to the amount of drug encapsulated into the microspheres, and W₂ refers to the total amount of drug administered. The evaluate results are shown in Fig. 4.

It can be seen from Fig. 4 that the encapsulation rate of the surface-functionalized PVA microspheres (i.e. A0 in Fig. 4) used in the control group, which is commonly used in the prior art, is 61%, and the encapsulation rate of the test group 1 (i.e. A2 in Fig. 4) is higher than that of test group 2 (i.e. A1 in Fig. 4), and the encapsulation rate of test group 1 using the microspheres capable of drug-loading of the present invention can be as high as 94%.

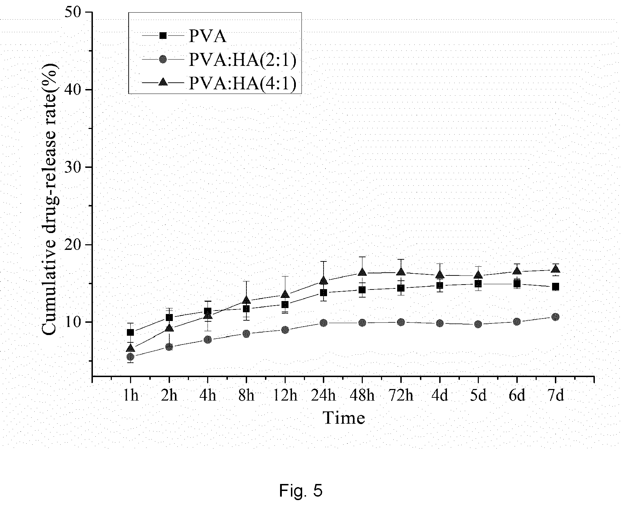

In a neutral pH environment, the controlled release effect of the microspheres in the control group and the experimental group 1 and the experimental group 2 were tested respectively. The drug release rate refers to the proportion of the drug released from the microspheres and the amount of drug packaged into the microspheres. Drug release rate=(W₃/W₁)×100%, W₃ is the amount of drug released from the microspheres. W₁ is the amount of drug packaged into the microspheres. The test results are shown in Fig. 5.

It can be seen from Fig. 5 that the drug release rate of the surface-functionalized PVA microspheres commonly used in the prior art in the control group and the drug release rate of the experimental group 2 approached 20% over time, while the drug release rate of the microspheres capable of drug-loading of the present invention in the experimental group 1 is stable at about 10%, showing good properties of sustained-release drugs. At the same time, it can be seen that the sustained-release effect of the microspheres capable of drug-loading is significantly better than that of the experimental group 1 (PVA:HA=2:1) compared with the experimental group 2 (PVA:HA=4:1). The preparation process parameter of the microspheres capable of drug-loading of the present invention is preferably PVA:HA=2:1.

From this example, it can be seen that microspheres capable of drug-loading of the present invention can achieve high encapsulation efficiency and sustained drug release characteristics by adding a certain proportion of HA particles to PVA. Based on the analysis of the structural characteristics of the microspheres capable of drug-loading of the present invention, the present invention pre-mixes HA particles and PVA with a certain proportion. The HA particles have a huge specific surface area, a porous structure, a high surface activity and other characteristics can make the drug slowly release from the internal pores. When the HA particles are in the solution, the hydroxyl groups on the surface will be vacant at a certain moment, and Ca²⁺ is exposed to form adsorption sites, which can be combined with functional groups such as hydroxyl and carboxyl groups in the drug molecule. Doxorubicin hydrochloride is rich in hydroxyl and amine groups and can be combined with Ca²⁺. At the same time, Ca²⁺ can also complex with the quinone and phenolic hydroxyl groups of doxorubicin hydrochloride. Therefore, compared with pure PVA microspheres, PVA/HA composite microspheres have higher drug loading and encapsulation efficiency.

In this example, only the microspheres capable of drug-loading prepared in Examples 2 and 3 are used as experiment objects, and the microspheres capable of drug-loading prepared in other examples have similar effects, which will not be repeated here.

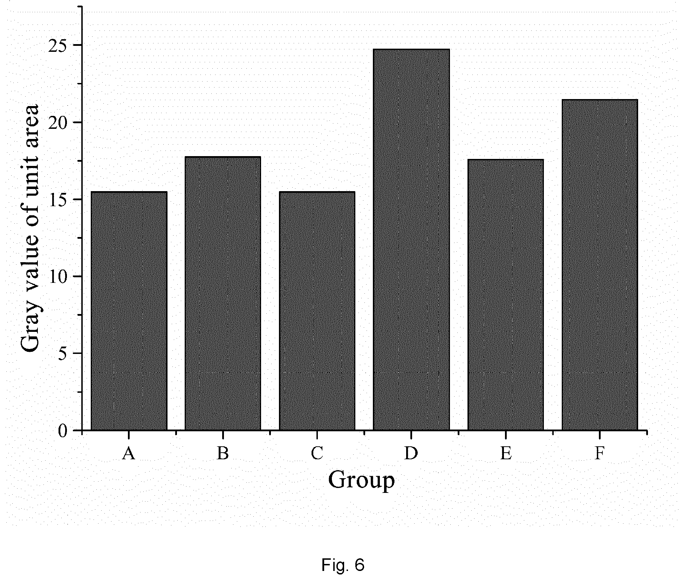

The radiopaque effect test of the microspheres capable of drug-loading of the present invention is as follows:
Test groups 1-6 were used for the radiopaque effect test. In Fig. 6, group A is sodium chloride saline, group B is dry PVA microspheres, group C is dry HA, group D is HA with saline, and group E is salt water and the microspheres described in Example 3 of the application (PVA:HA=4:1), and group F is added salt water and the microspheres described in Example 2 of the application (PVA:HA=2:1). The X-ray imaging conditions of the test groups 1-6 were tested respectively, and the test results are shown in Fig. 6. The group E and group F have obvious radiopaque effects, indicating that the microspheres capable of drug-loading of the present invention have a certain radiopaque effect, and the radiopaque effect of group F is higher than that of group B and group C.

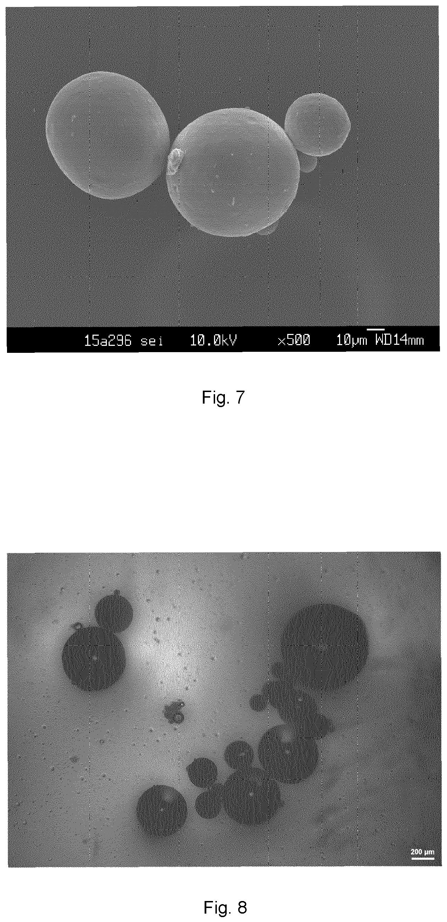

Figure 7 is a scanning electron micrograph of the microspheres capable of drug-loading according to the embodiments of the present invention. It can be seen that microspheres are smooth in shape without too many small irregular particle sizes. The microspheres with such a structure have a huge specific surface area, a porous structure, and a high surface activity, and possess good combination with drugs, can greatly increase the drug loading, and make the embolization effect perfect. Fig. 8 is a microscopic photograph of the microspheres capable of drug-loading according to an embodiment of the present invention. It can be seen that microspheres are difficult to transmit light and have a certain radiopaque effect, which is better than the PVA/HA composite microspheres prepared by the method in the prior art.

In the preparation method of the present invention, alkaline solution is not added in Step 2, but glutaraldehyde is added to react for 3 to 30 seconds, and the reaction liquid is added dropwise to the mixture oil phase before the reaction liquid is coagulated, so that the reaction time is precisely controlled, making the microspheres capable of drug-loading of the present invention can have a certain degree of radiopaque effect.

At present, solid embolic materials are mainly used in the interventional clinical treatment of liver cancer. There are many commercial microspheres on the market, but they usually do not have a good radiopaque effect. The developed image cannot truly reflect the real-time position of the microspheres, which affects the monitoring of embolism.

The present application uses HA particles and PVA to prepare microspheres, both of which have good biocompatibility and non-biological toxicity, are used in medical treatment, and have good embolization effects. The HA particles are embedded in PVA after the polymerization reaction and will not escape, which can improve the development effect and is safe and reliable.

In this example, only the microspheres capable of drug-loading prepared in Examples 2 and 3 are used as experiment objects, and the microspheres capable of drug-loading prepared in other examples have similar effects, which will not be repeated here.

## Claims

1. A preparation method of microspheres capable of drug-loading, comprising the following steps:
(1) Mix an aqueous solution of poly(vinyl alcohol) (PVA) and hydroxyapatite (HA) particles evenly, wherein the mass ratio of the PVA to water is 0.2:100 to 10:100;
(2) Add concentrated hydrochloric acid to the evenly mixture solution in Step (1), and then add glutaraldehyde to obtain a reaction solution, after pre-reacting for 3 to 30 seconds, the pre-reaction solution is added dropwise into a mixture oil phase before the pre-reaction solution is coagulated, stir and react in an oil bath at 50°C-60°C; the mixture oil phase includes liquid paraffin and sorbitan fatty acid ester;
(3) Collect the reaction mixture in Step (2), filter, wash, and dry to obtain microspheres capable of drug-loading.

2. The method for preparing microspheres capable of drug-loading according to claim 1, wherein the mass ratio of PVA to HA particles in Step (1) is 1:1-10:1.

3. The method for preparing microspheres capable of drug-loading according to claim 1 or 2, wherein the mass ratio of PVA to HA particles in Step (1) is 1.5:1 to 4:1.

4. The method for preparing microspheres capable of drug-loading according to claim 1, wherein the mass ratio of glutaraldehyde to PVA added in Step (2) is 1:50-1:20; and the mass ratio of PVA to liquid paraffin in the mixture oil phase in Step (2) is 1:1-1:1.5.

5. The method for preparing microspheres capable of drug-loading according to claim 1, wherein in the mixture oil phase of Step (2), the mass ratio of liquid paraffin to sorbitan fatty acid ester is 50:1-100:1.

6. The method for preparing microspheres capable of drug-loading according to claim 1, wherein in the Step (3), a lyophilizer or a dryer is used for drying.

## Patentansprüche

1. Herstellungsverfahren von Mikrosphären, die zu einer Arzneimittelbeladung in der Lage sind, umfassend die folgenden Schritte:
(1) gleichmäßiges Mischen einer wässrigen Lösung von Partikeln aus Poly(vinylalkohol) (PVA) und Hydroxyapatit (HA), wobei das Massenverhältnis des PVA zu Wasser 0,2:100 bis 10:100 beträgt;
(2) Zugeben von konzentrierter Salzsäure zu der gleichmäßigen Mischungslösung in Schritt (1), und anschließendes Zugeben von Glutaraldeyhd, um eine Reaktionslösung zu erhalten, nach Vor-Umsetzen während 3 bis 30 Sekunden, wobei die Vor-Reaktionslösung tropfenweise in eine Mischungsölphase zugegeben wird, bevor die Vor-Reaktionslösung koaguliert wird, Rühren und Umsetzen in einem Ölbad bei 50 °C bis 60 °C; wobei die Mischungsölphase flüssiges Paraffin und Sorbitanfettsäureester aufweist;
(3) Sammeln der Reaktionsmischung in Schritt (2), Filtrieren, Waschen und Trocknen, um Mikrosphären zu erhalten, die zu einer Arzneimittelbeladung in der Lage sind.

2. Herstellungsverfahren von Mikrosphären, die zu einer Arzneimittelbeladung in der Lage sind, nach Anspruch 1, wobei das Massenverhältnis von PVA- zu HA-Partikeln in Schritt (1) 1:1 bis 10:1 beträgt.

3. Herstellungsverfahren von Mikrosphären, die zu einer Arzneimittelbeladung in der Lage sind, nach Anspruch 1 oder 2, wobei das Massenverhältnis von PVA- zu HA-Partikeln in Schritt (1) 1,5:1 bis 4:1 beträgt.

4. Herstellungsverfahren von Mikrosphären, die zu einer Arzneimittelbeladung in der Lage sind, nach Anspruch 1, wobei das Massenverhältnis von Glutaraldehd zu PVA, der in Schritt (2) zugegeben wird, 1:50 bis 1:20 beträgt, und das Massenverhältnis von PVA zu flüssigem Paraffin in der Mischungsölphase in Schritt (2) 1:1 bis 1:1,5 beträgt.

5. Herstellungsverfahren von Mikrosphären, die zu einer Arzneimittelbeladung in der Lage sind, nach Anspruch 1, wobei in der Mischungsölphase von Schritt (2) das Massenverhältnis von flüssigem Paraffin zu Sorbitanfettsäurester 50:1 bis 100:1 beträgt.

6. Herstellungsverfahren von Mikrosphären, die zu einer Arzneimittelbeladung in der Lage sind, nach Anspruch 1, wobei in dem Schritt (3) ein Lyophilisator oder ein Trockner zum Trocknen verwendet wird.

## Revendications

1. Procédé de préparation de microsphères capables de chargement de médicaments, comprenant les étapes suivantes :
(1) mélange d'une solution aqueuse d'alcool polyvinylique (PVA) et de particules d'hydroxyapatite (HA) de manière homogène, dans lequel le rapport de masse du PVA sur l'eau est de 0,2/100 à 10/100 ;
(2) ajout d'acide chlorhydrique concentré à la solution mélangée de manière homogène de l'étape (1), puis ajout de glutaraldéhyde pour obtenir une solution de réaction, après une pré-réaction de 3 à 30 secondes, ajout de la solution de pré-réaction goutte à goutte dans une phase huileuse mélangée avant la coagulation de la solution de pré-réaction, agitation et mise en réaction dans un bain d'huile à 50 °C à 60 °C ; la phase huileuse mélangée inclut de la paraffine liquide et un ester d'acide gras de sorbitane ;
(3) collecte du mélange réactionnel de l'étape (2), filtration, lavage, et séchage pour obtenir des microsphères capables de chargement de médicaments.

2. Procédé de préparation de microsphères capables de chargement de médicaments selon la revendication 1, dans lequel le rapport de masse du PVA sur les particules de HA à l'étape (1) est de 1/1 à 10/1.

3. Procédé de préparation de microsphères capables de chargement de médicaments selon la revendication 1 ou 2, dans lequel le rapport de masse du PVA sur les particules de HA à l'étape (1) est de 1,5/1 à 4/1.

4. Procédé de préparation de microsphères capables de chargement de médicaments selon la revendication 1, dans lequel le rapport de masse du glutaraldéhyde sur le PVA ajouté à l'étape (2) est de 1/50 à 1/20 ; et le rapport de masse du PVA sur la paraffine liquide dans la phase huileuse mélangée à l'étape (2) est de 1/1 à 1/1,5.

5. Procédé de préparation de microsphères capables de chargement de médicaments selon la revendication 1, dans lequel dans la phase huileuse mélangée de l'étape (2), le rapport de masse de la paraffine liquide sur l'ester d'acide gras de sorbitane est de 50/1 à 100/1.

6. Procédé de préparation de microsphères capables de chargement de médicaments selon la revendication 1, dans lequel à l'étape (3), un lyophilisateur ou un séchoir est utilisé pour le séchage.
